# EUROPEAN PATENT APPLICATION

(11) **EP 4 656 206 A1**
(43) Date of publication of application: **03.12.2025**
(21) Application number: 23918433.6
(22) Date of filing: 27.01.2023
(51) Int. Cl.: A61K 45/00, A61K 9/70, A61K 47/06, A61K 47/10

(54) **NON-AQUEOUS PERCUTANEOUSLY ABSORPTIVE FORMULATION**

(71) Applicant: Lead Chemical Co., Ltd., Toyama-shi, Toyama 930-0912 (JP)
(72) Inventor: MURAI, Naoki, Toyama-shi, Toyama 930-0912 (JP); WAKI, Hitomi, Toyama-shi, Toyama 930-0912 (JP); FUKAO, Miki, Toyama-shi, Toyama 930-0912 (JP)
(74) Representative: Geling, Andrea
(86) International application number: PCT/JP2023/002714
(87) International publication number: WO 2024/157473

(57) **Abstract**

Provided is a nonaqueous preparation for percutaneous absorption that sufficiently exhibits its medicinal effects and has sufficient adhesion and manufacturing suitability despite the amount of a drug in an adhesive layer constituting the nonaqueous preparation for percutaneous absorption being small (2% by mass to 4% by mass). A nonaqueous preparation for percutaneous absorption includes a support and an adhesive layer laminated on the support. The adhesive layer contains: a non-steroidal anti-inflammatory drug in an alkali metal salt form in an amount of 2% by mass to 4% by mass based on a total mass of the adhesive layer; a rosin ester derivative in an amount of 10% by mass to 30% by mass based on the total mass of the adhesive layer; and an inorganic acid.

## Description

### TECHNICAL FIELD

The present invention relates to a nonaqueous preparation for percutaneous absorption. More specifically, the present invention relates to a nonaqueous preparation for percutaneous absorption containing a non-steroidal anti-inflammatory drug.

### BACKGROUND ART

Non-steroidal anti-inflammatory drugs do not show severe side effects as shown in steroidal anti-inflammatory analgesics, and thus are widely used in the clinical practice. However, in a case where non-steroidal anti-inflammatory drugs are orally administered, they show any inhibition activity against cyclooxygenase being a prostaglandin generating enzyme present in the living bodies, leading to the manifestation of side effects such as stomach mucosa disorder. In order to reduce such side effects, preparations that allow drugs to be absorbed through the skin, that is, transdermal preparations, are developed.

Transdermal preparations are broadly divided into matrix and reservoir types. A matrix-type transdermal preparation is constituted of an adhesive layer containing a drug and a support that retains the adhesive layer. However, with the exception of a very few drugs, many drugs do not allow their medicinal effects to be produced when they are applied to the skin alone, and measures to improve the skin penetration of drugs have been taken. For example, there has been proposed a nonaqueous preparation for percutaneous absorption that contains a non-steroidal anti-inflammatory drug in an alkali metal salt form in combination with an inorganic acid which is strongly acidic compared with the non-steroidal anti-inflammatory drug in a free form, thereby improving the drug-releasing characteristics and skin penetration of the non-steroidal anti-inflammatory drug (Patent Document 1).
Patent Document 1: Japanese Patent No. 4678532

### DISCLOSURE OF THE INVENTION

### PROBLEMS TO BE SOLVED BY THE INVENTION

Generally, it is believed that the higher the content of a drug in the adhesive layer constituting a transdermal preparation, the higher the skin penetration of the drug. The nonaqueous preparation for percutaneous absorption proposed in Patent Document 1, which improves the skin penetration of a drug, contains 5% by mass loxoprofen sodium, 0.5% by mass phosphoric acid, and 10% by mass rosin glycerin ester (Example 2 of Patent Document 1).

In addition, it is believed that the lower the content of non-drug components in the adhesive layer constituting the transdermal preparation, such as a tackifier (a rosin ester derivative and the like), the more easily the drug can penetrate the skin, increasing the skin penetration of the drug. However, if, for example, the content of the tackifier is low, the transdermal preparation does not have sufficient adhesion, making it difficult to satisfy the required performance. Further, since the adhesive layer forming composition does not have viscosity suitable for industrial manufacturing, the transdermal preparation is difficult to satisfy manufacturing suitability that allows it to be manufactured by an industrial manufacturing method (hereinafter also referred to as "manufacturing suitability").

An object of the present invention is to provide a nonaqueous preparation for percutaneous absorption that sufficiently exhibits its medicinal effects and has sufficient adhesion and manufacturing suitability despite the amount of a drug in an adhesive layer constituting the nonaqueous preparation for percutaneous absorption being small (2% by mass to 4% by mass).

### SOLUTIONS TO THE PROBLEMS

The present inventors eagerly studied in order to solve the above-described problems. Consequently, the present inventors found that by containing a 2% by mass to 4% by mass non-steroidal anti-inflammatory drug in an alkali metal salt form in combination with a 10% by mass to 30% by mass rosin ester derivative in an adhesive layer constituting a nonaqueous preparation for percutaneous absorption, the non-steroidal anti-inflammatory drug can sufficiently exhibits its medicinal effects, that is, excellent skin penetration of a non-steroidal anti-inflammatory drug can develop, and the nonaqueous preparation for percutaneous absorption including the adhesive layer has sufficient adhesion and manufacturing suitability, despite the amount of the non-steroidal anti-inflammatory drug being small (2% by mass to 4% by mass), and they completed the present invention.

That is, the present invention relates to a nonaqueous preparation for percutaneous absorption including a support and an adhesive layer laminated on the support, in which
the adhesive layer contains:
a non-steroidal anti-inflammatory drug in an alkali metal salt form in an amount of 2% by mass to 4% by mass based on a total mass of the adhesive layer;
a rosin ester derivative in an amount of 10% by mass to 30% by mass based on the total mass of the adhesive layer; and
an inorganic acid.

Preferred embodiments of the present invention include:
the nonaqueous preparation for percutaneous absorption in which the non-steroidal anti-inflammatory drug is loxoprofen sodium hydrate, and the rosin ester derivative is hydrogenated rosin glycerin ester;
the nonaqueous preparation for percutaneous absorption in which the inorganic acid is phosphoric acid;
the nonaqueous preparation for percutaneous absorption in which the adhesive layer contains a rubbery adhesive, and the rubbery adhesive is a styrene-isoprene-styrene block copolymer;
the nonaqueous preparation for percutaneous absorption in which the adhesive layer further contains at least one selected from the group consisting of a plasticizer, a tackifier, a percutaneous absorption accelerator, and a stabilizer; and
the nonaqueous preparation for percutaneous absorption in which the nonaqueous preparation for percutaneous absorption further comprises a liner layer laminated on the adhesive layer.

### EFFECTS OF THE INVENTION

With the present invention, a nonaqueous preparation for percutaneous absorption that has excellent skin penetration of a non-steroidal anti-inflammatory drug and has sufficient adhesion and manufacturing suitability despite the amount of a drug in an adhesive layer constituting the nonaqueous preparation for percutaneous absorption being small (2% by mass to 4% by mass) can be provided.

### BRIEF DESCRIPTION OF THE DRAWINGS

Fig. 1 is a graph showing the relationship between the content of loxoprofen sodium and the content of hydrogenated rosin glycerin ester (10.0% by mass) in the results of a silicone membrane penetration test of Test Example 1.
Fig. 2 is a graph showing the relationship between the content of the loxoprofen sodium and the content of the hydrogenated rosin glycerin ester (20.0% by mass) in the results of the silicone membrane penetration test of Test Example 1.
Fig. 3 is a graph showing the relationship between the content of the loxoprofen sodium (2.0% by mass) and the content of the hydrogenated rosin glycerin ester in the results of the silicone membrane penetration test of Test Example 1.
Fig. 4 is a graph showing the relationship between the content of the loxoprofen sodium (3.0% by mass) and the content of the hydrogenated rosin glycerin ester in the results of the silicone membrane penetration test of Test Example 1.
Fig. 5 is a graph showing the relationship between the content of the loxoprofen sodium (4.0% by mass) and the content of the hydrogenated rosin glycerin ester in the results of the silicone membrane penetration test of Test Example 1.

### DESCRIPTION OF PREFERRED EMBODIMENTS

The present invention relates to a nonaqueous preparation for percutaneous absorption including a support and an adhesive layer laminated on the support, in which
the adhesive layer contains:
a non-steroidal anti-inflammatory drug in an alkali metal salt form in an amount of 2% by mass to 4% by mass based on the total mass of the adhesive layer;
a rosin ester derivative in an amount of 10% by mass to 30% by mass based on the total mass of the adhesive layer; and
an inorganic acid.

### [Adhesive Layer]

The adhesive layer contains a specific amount of a non-steroidal anti-inflammatory drug in an alkali metal salt form, a specific amount of a rosin ester derivative, and an inorganic acid.

Although the thickness of the adhesive layer is not specifically limited, it is, for example, 35 µm to 400 µm, preferably 75 µm to 250 µm, and more preferably 100 µm to 200 µm.

In addition, from the viewpoint of the maintenance of adhesion and the property of following skin, the mass of the adhesive layer of the nonaqueous preparation for percutaneous absorption is, for example, 0.3 g/70 cm² or more, preferably 0.5 g/70 cm² or more, and more preferably 0.7 g/70 cm² or more.

### [Adhesive]

An adhesive is a component that imparts adhesion to the adhesive layer.

The adhesive is not specifically limited, and examples include rubbery adhesives, acrylic adhesives, and silicone adhesives.

The rubbery adhesives are not specifically limited, and examples include synthetic rubber, such as polyisoprene, polyisobutylene, styrene-isoprene-styrene block copolymers (SIS), styrene-butadiene-styrene block copolymers (SBS), and styrene-butadiene rubber (SBR), and natural rubber.

The acrylic adhesives are not specifically limited, and examples include those obtained through polymerization or copolymerization of at least one of alkyl (meth)acrylate monomers, such as ethyl (meth)acrylate, butyl (meth)acrylate, 2-ethylhexyl (meth)acrylate, and isononyl (meth)acrylate. In the description, (meth)acrylate means acrylate and/or methacrylate.

The silicone adhesives are not specifically limited, and examples include those containing silicone rubber, such as polydimethylsiloxane, polymethylvinylsiloxane, and polymethylphenylsiloxane, and the like as a main component.

In the present invention, one adhesive may be used alone, or two or more adhesives may be used in combination.

In the present invention, as the adhesive, the rubbery adhesives are preferred, at least one selected from the group consisting of styrene-isoprene-styrene block copolymers (SIS) and polyisobutylene is more preferred, and using a styrene-isoprene-styrene block copolymer (SIS) and polyisobutylene in combination is further preferred.

In the present invention, the content of the adhesive is usually 10% by mass to 50% by mass, preferably 15% by mass to 40% by mass, and further preferably 20% by mass to 35% by mass, based on the total mass of the adhesive layer in the nonaqueous preparation for percutaneous absorption.

### <Non-steroidal anti-inflammatory drug in Alkali Metal Salt Form>

In the present invention, the non-steroidal anti-inflammatory drug in an alkali metal salt form is a component that exerts anti-inflammatory and analgesic actions.

The non-steroidal anti-inflammatory drug is not specifically limited, and examples include propionic acid-based non-steroidal anti-inflammatory drugs, phenylacetic acid-based non-steroidal anti-inflammatory drugs, fenamic acid-based non-steroidal anti-inflammatory drugs, and acetic acid-based non-steroidal anti-inflammatory drugs.

Examples of the alkali metal include, for example, sodium and calcium.

Examples of the non-steroidal anti-inflammatory drug in an alkali metal salt form include loxoprofen sodium hydrate, diclofenac sodium, tolmetin sodium, sodium meclofenamate, sodium meclofenamate hydrate, amfenac sodium hydrate, and zomepirac sodium hydrate.

In the present invention, one non-steroidal anti-inflammatory drug in an alkali metal salt form may be used alone, or two or more non-steroidal anti-inflammatory drugs in an alkali metal salt form may be used in combination.

In the present invention, as the non-steroidal anti-inflammatory drug in an alkali metal salt form, the propionic acid-based non-steroidal anti-inflammatory drugs and the phenylacetic acid-based non-steroidal anti-inflammatory drugs are preferred, loxoprofen sodium hydrate and diclofenac sodium are more preferred, and loxoprofen sodium hydrate is particularly preferred.

In the present invention, the content of the non-steroidal anti-inflammatory drug in an alkali metal salt form is 2% by mass to 4% by mass based on the total mass of the adhesive layer in the nonaqueous preparation for percutaneous absorption. When the non-steroidal anti-inflammatory drug is a hydrate, the content of the non-steroidal anti-inflammatory drug is calculated on an anhydrous basis. In a case where the content of the non-steroidal anti-inflammatory drug in an alkali metal salt form is less than 2% by mass or more than 4% by mass, the cumulative penetration amount of the non-steroidal anti-inflammatory drug for 24 hours decreases rapidly.

### <Rosin Ester Derivative>

In the present invention, the rosin ester derivative is a component that develops sufficient adhesion and manufacturing suitability.

The rosin ester derivative is not specifically limited, and examples include rosin glycerin ester, hydrogenated rosin glycerin ester, rosin pentaerythritol ester, and hydrogenated rosin pentaerythritol ester.

One rosin ester derivative may be used alone, or two or more rosin ester derivatives may be used in a combination.

In the present invention, as the rosin ester derivative, hydrogenated rosin glycerin ester is preferred.

In the present invention, the content of the rosin ester derivative is 10% by mass to 30% by mass based on the total mass of the adhesive layer in the nonaqueous preparation for percutaneous absorption. In a case where the content of the rosin ester derivative is less than 10% by mass, sufficient adhesion and manufacturing suitability do not develop. In addition, in a case where the content is more than 30% by mass, the cumulative penetration amount of the non-steroidal anti-inflammatory drug in an alkali metal salt form for 24 hours decreases rapidly.

### <Inorganic Acid>

In the present invention, the inorganic acid is a component that exerts an action to improve the solubility of the non-steroidal anti-inflammatory drug in an alkali metal salt form in the adhesive.

The inorganic acid is preferably strongly acidic compared with the non-steroidal anti-inflammatory drug in a free form, and examples include pharmaceutically acceptable inorganic acids, such as phosphoric acid, hydrochloric acid, nitric acid, and sulfuric acid.

One inorganic acid may be used alone, or two or more inorganic acids may be used in combination

In the present invention, as the inorganic acid, phosphoric acid is preferred.

In the present invention, the content of the inorganic acid is, for example, 0.01% by mass to 20% by mass, preferably 0.05% by mass to 10% by mass, and further preferably 0.1% by mass to 5% by mass, based on the total mass of the adhesive layer in the nonaqueous preparation for percutaneous absorption.

### <Other Additives>

The adhesive layer in the nonaqueous preparation for percutaneous absorption of the present invention may include a plasticizer, a tackifier, a percutaneous absorption accelerator, a stabilizer such as an antioxidant and a UV absorber, a filler, a crosslinking agent, an antiseptic agent, and the like, to the extent that the effect of the present invention is not impaired. One of these may be used alone, or two or more of these may be used in combination.

The plasticizer is not specifically limited, and examples include petroleum oils (for example, paraffin process oil such as liquid paraffin, naphthene process oil, and aromatic process oil), squalane, squalene, vegetable oils (for example, olive oil, camellia oil, castor oil, tall oil, and earthnut oil), silicone oil, dibasic acid esters (for example, dibutyl phthalate and dioctyl phthalate), liquid rubber (for example, liquid polybutene and liquid isoprene rubber), fatty acid esters (for example, isopropyl myristate, hexyl laurate, diethyl sebacate, and diisopropyl sebacate), diethylene glycol, polyethylene glycol, glycol salicylate, propylene glycol, dipropylene glycol, triacetin, triethyl citrate, and crotamiton. One plasticizer may be used alone, or two or more plasticizers may be used in combination.

In the present invention, as the plasticizer, at least one selected from the group consisting of liquid paraffin, isopropyl myristate, and crotamiton is preferred, and liquid paraffin is more preferred.

The content of the plasticizer is, for example, 5% by mass to 70% by mass, preferably 10% by mass to 65% by mass, and more preferably 10% by mass to 60% by mass, based on the total mass of the adhesive layer. In a case where the content of the plasticizer is less than 5% by mass, the effect of improvement in the cohesion of the adhesive layer by the plasticizer may not sufficiently develop. On the other hand, in a case where it is more than 70% by mass, the skin penetration of the non-steroidal anti-inflammatory drug in an alkali metal salt form may be inhibited.

The tackifier is not specifically limited, and examples include alicyclic saturated hydrocarbon resins, aliphatic hydrocarbon resins, and terpene resins. One tackifier may be used alone, or two or more tackifiers may be used in combination.

In the present invention, as the tackifier, at least one selected from the group consisting of aliphatic hydrocarbon resins and terpene resins is preferred, and aliphatic hydrocarbon resins are more preferred.

The content of the tackifier is, for example, 3% by mass to 60% by mass, preferably 5% by mass to 50% by mass, and more preferably 7% by mass to 40% by mass, based on the total mass of the adhesive layer. In a case where the content of the tackifier is less than 3% by mass, the effect of improvement in the adhesion of the adhesive layer by the tackifier may not sufficiently develop. On the other hand, in a case where it is more than 60% by mass, skin irritation on peeling off of the nonaqueous preparation for percutaneous absorption tends to increase.

In the present invention, the percutaneous absorption accelerator is not specifically limited, and examples include organic acids (for example, cinnamic acid and salicylic acid), organic acid esters (for example, methyl cinnamate, methyl salicylate, ethylene glycol salicylate, cetyl lactate, lauryl lactate, ethyl acetate, and propyl acetate), fatty acids having 8 to 20 carbon atoms (for example, caproic acid, caprylic acid, capric acid, lauric acid, myristic acid, palmitic acid, stearic acid, isostearic acid, oleic acid, linoleic acid, and linolenic acid), fatty acid esters having 8 to 20 carbon atoms (for example, methyl laurate, hexyl laurate, isopropyl myristate, myristyl myristate, octyldecyl myristate, and cetyl palmitate), aliphatic alcohol (for example, lauryl alcohol, myristyl alcohol, cetyl alcohol, isostearyl alcohol, and oleyl alcohol), monoterpene-based compounds (for example, 1-menthol, geraniol, thymol, eugenol, terpineol, borneol, d-limonene, isoeugenol, isoborneol, nerol, and dl-camphor), monoglyceryl fatty acid esters (for example, glyceryl monocaprylate, glyceryl monocaprate, glyceryl monolaurate, and glyceryl monoleate), sorbitan fatty acid esters (for example, sorbitan monolaurate), sucrose fatty acid esters (for example, sucrose monolaurate ester), polysorbates (for example, polysorbate 20), polyhydric alcohol (for example, propylene glycol), propylene glycol fatty acid esters (for example, propylene glycol monolaurate), polyethylene glycol fatty acid esters (for example, polyethylene glycol monolaurate and polyethylene glycol monostearate), polyoxyethylene alkyl ethers (for example, polyoxyethylene lauryl ether), polyoxyethylene-cured castor oils (for example, polyoxyethylene-cured castor oil), and pyrrolidones (for example, pyrrothiodecane). One percutaneous absorption accelerator may be used alone, or two or more percutaneous absorption accelerators may be used in combination.

In the present invention, as the percutaneous absorption accelerator, at least one selected from the group consisting of aliphatic alcohol, monoterpene-based compounds, monoglyceryl fatty acid esters, sorbitan fatty acid esters, propylene glycol fatty acid esters, polyoxyethylene alkyl ethers, and pyrrolidones is preferred, and at least one selected from the group consisting of lauryl alcohol, myristyl alcohol, isostearyl alcohol, l-menthol, glyceryl monocaprylate, glyceryl monocaprate, glyceryl monoleate, sorbitan monolaurate, propylene glycol monolaurate, polyoxyethylene lauryl ether, and pyrrothiodecane is more preferred.

The content of the percutaneous absorption accelerator is, for example, 0.01% by mass to 20% by mass, preferably 0.05% by mass to 10% by mass, and more preferably 0.1% by mass to 5% by mass, based on the total mass of the adhesive layer. In a case where the content of the percutaneous absorption accelerator is less than 0.01% by mass, the effect of improvement in the skin penetration of the non-steroidal anti-inflammatory drug in an alkali metal salt form by the percutaneous absorption accelerator may not sufficiently develop. On the other hand, in a case where it is more than 20% by mass, skin irritation such as edema tends to increase, and further, adherence to the skin tends to decrease.

The antioxidant is not specifically limited, and examples include tocopherol and its ester derivatives, ascorbic acid, ascorbyl stearate ester, nordihydroguaiaretic acid, dibutyl hydroxytoluene (BHT), and butyl hydroxyanisole (BHA). One antioxidant may be used alone, or two or more antioxidants may be used in combination.

The content of the antioxidant is, for example, 0.01% by mass to 10% by mass, preferably 0.05% by mass to 5% by mass, and more preferably 0.1% by mass to 2% by mass, based on the total mass of the adhesive layer.

The UV absorber is not specifically limited, and examples include p-aminobenzoic acid derivatives, anthranilic acid derivatives, salicylic acid derivatives, coumarin derivatives, amino acid compounds, imidazoline derivatives, benzophenone derivatives, cinnamic acid derivatives, pyrimidine derivatives, and oxane derivatives. One UV absorber may be used alone, or two or more UV absorbers may be used in combination.

The content of the UV absorber is, for example, 0.01% by mass to 10% by mass, preferably 0.05% by mass to 5% by mass, and more preferably 0.1% by mass to 2% by mass, based on the total mass of the adhesive layer.

The filler is not specifically limited, and examples include calcium carbonate, magnesium carbonate, soft anhydrous silicic acid, hydrous silicon dioxide or silicates (for example, aluminum silicate and magnesium silicate), barium sulfate, calcium sulfate, calcium zincate, zinc oxide, titanium oxide, bentonite, kaolin, and talc. One filler may be used alone, or two or more fillers may be used in combination.

The content of the filler is, for example, 0.01% by mass to 10% by mass, preferably 0.05% by mass to 5% by mass, and more preferably 0.1% by mass to 2% by mass, based on the total mass of the adhesive layer.

The crosslinking agent is not specifically limited, and examples include thermoset resins (for example, amino resins, phenol resins, epoxy resins, alkyd resins, and unsaturated polyesters), isocyanate compounds, blocked isocyanate compounds, organic crosslinking agents (for example, organic peroxide), and inorganic crosslinking agents (for example, metals and metal compounds). One crosslinking agent may be used alone, or two or more crosslinking agents may be used in combination.

The content of the crosslinking agent is, for example, 0.01% by mass to 10% by mass, preferably 0.05% by mass to 5% by mass, and more preferably 0.1% by mass to 2% by mass, based on the total mass of the adhesive layer.

The antiseptic agent is not specifically limited, and examples include thymol, isopropyl methylphenol, benzoic acid and its salts, sorbic acid and its salts, methyl parahydroxybenzoate, ethyl parahydroxybenzoate, propyl parahydroxybenzoate, butyl parahydroxybenzoate, benzyl alcohol, benzalkonium chloride, and benzethonium chloride. One antiseptic agent may be used alone, or two or more antiseptic agents may be used in combination.

The content of the antiseptic agent is, for example, 0.01% by mass to 10% by mass, preferably 0.05% by mass to 5% by mass, and more preferably 0.1% by mass to 2% by mass, based on the total mass of the adhesive layer.

### [Support]

The support is not specifically limited, and, for example, woven fabrics, unwoven fabrics, knitting fabrics, resin films, paper, and their laminates can be used.

The material of the support is not specifically limited, and examples include polyesters such as polyethylene terephthalate and polybutylene terephthalate, polyolefins such as polyethylene and polypropylene, and synthetic resins such as polyvinyl chloride, polycarbonate, polyurethane, and cellophane. Among them, a polyethylene terephthalate knitting fabric and a polyethylene terephthalate unwoven fabric are preferred as the support.

Although the thickness of the support is not specifically limited, it is, for example, 5 µm to 1000 µm, preferably 50 µm to 800 µm, and more preferably 400 µm to 600 µm. In a case where the thickness of the support is less than 5 µm, handling ease for affixing the nonaqueous preparation for percutaneous absorption tends to decrease. On the other hand, in a case where it is more than 1000 µm, the ease of manufacturing tends to decrease, for example, cutting the support or the adhesive layer becomes difficult in the manufacturing process of the nonaqueous preparation for percutaneous absorption.

### [Liner Layer]

The nonaqueous preparation for percutaneous absorption of the present invention may have a liner layer laminated on the above-described adhesive layer. By laminating the liner layer, the adhesive layer can be protected until the use of the nonaqueous preparation for percutaneous absorption.

The liner layer is not specifically limited, and examples include films made of materials, including polyesters such as polyethylene terephthalate and polybutylene terephthalate, polyolefins such as polyethylene and polypropylene, polyvinyl chloride, polyamides such as nylon, polyurethane, aluminum foil, and paper, and laminates of these films. Among them, a polyethylene terephthalate film is preferred as the liner layer.

In addition, silicone treatment or fluorine treatment may be performed on the surface on the side of the liner layer, which is in contact with the adhesive layer. This makes it easier to peel off the liner layer from the adhesive layer when the nonaqueous preparation for percutaneous absorption is used.

Although the thickness of the liner layer is not specifically limited, it is, for example, 35 µm to 200 µm, preferably 50 µm to 100 µm.

The method for manufacturing the nonaqueous preparation for percutaneous absorption of the present invention is not specifically limited. Examples of the manufacturing method include, for example, a method in which the adhesive layer is formed by dissolving the non-steroidal anti-inflammatory drug in an alkali metal salt form, the rosin ester derivative, the inorganic acid, and optional components as needed in a solvent (such as toluene, xylene, ethyl acetate, butyl acetate, hexane, heptane, cyclohexane, ethanol, methanol, isopropanol) to prepare an application solution and applying the application solution on the support to vaporize the solvent in the application solution, and a method in which the adhesive layer is formed by heating and melting the non-steroidal anti-inflammatory drug in an alkali metal salt form, the rosin ester derivative, the inorganic acid, and optional components as needed and spreading this molten product on the support. Furthermore, the nonaqueous preparation for percutaneous absorption including a liner layer can be manufactured by forming the liner layer on the adhesive layer by, for example, laminating the adhesive layer and the liner layer together.

### EXAMPLES

Hereinafter, the present invention will be described more specifically based on Examples. However, the present invention is not limited to them in any way.

### [Examples]

Nonaqueous preparations for percutaneous absorption with the compositions shown in Table 1-1 and Table 1-2 were manufactured. Specifically, hydrogenated rosin glycerin ester, a styrene-isoprene-styrene block copolymer, polyisobutylene, liquid paraffin, and dibutyl hydroxytoluene were melted and kneaded at about 150°C, and then, loxoprofen sodium hydrate, phosphoric acid, and 1-menthol were added and mixed thereto to obtain an adhesive layer forming composition. The obtained adhesive layer forming composition was spread on a release-treated surface of a 75 µm thick polyethylene terephthalate film to have a mass of 1.0 g/70 cm², forming an approximately 150 µm thick adhesive layer. Next, a 500 µm thick polyethylene terephthalate knitting fabric was laminated onto the adhesive layer, and they were cut in a desired size to obtain a nonaqueous preparation for percutaneous absorption.

The amounts shown in Table 1-1 and Table 1-2 are in % by mass.

**[Table 1-1]**

| | Example 1 | Example 2 | Example 3 | Example 4 | Example 5 | Example 6 |
|---|---|---|---|---|---|---|
| Loxoprofen sodium hydrate | 2.3 | 2.3 | 2.3 | 3.4 | 3.4 | 3.4 |
| Hydrogenated rosin glycerin ester | 10.0 | 20.0 | 30.0 | 10.0 | 15.0 | 20.0 |
| Phosphoric acid | 1.0 | 1.0 | 1.0 | 1.0 | 1.0 | 1.0 |
| Styrene-isoprene-styrene block copolymer | 22.5 | 22.5 | 22.5 | 22.5 | 22.5 | 22.5 |
| Polyisobutylene | 7.5 | 7.5 | 7.5 | 7.5 | 7.5 | 7.5 |
| Liquid paraffin | 53.2 | 43.2 | 33.2 | 52.1 | 47.1 | 42.1 |
| 1-menthol | 3.0 | 3.0 | 3.0 | 3.0 | 3.0 | 3.0 |
| Dibutyl hydroxytoluene | 0.5 | 0.5 | 0.5 | 0.5 | 0.5 | 0.5 |
| Total | 100.0 | 100.0 | 100.0 | 100.0 | 100.0 | 100.0 |

**[Table 1-2]**

| | Example 7 | Example 8 | Example 9 | Example 10 | Example 11 | Example 12 |
|---|---|---|---|---|---|---|
| Loxoprofen sodium hydrate | 3.4 | 3.4 | 4.5 | 4.5 | 4.5 | 4.5 |
| Hydrogenated rosin glycerin ester | 25.0 | 30.0 | 10.0 | 20.0 | 25.0 | 30.0 |
| Phosphoric acid | 1.0 | 1.0 | 1.0 | 1.0 | 1.0 | 1.0 |
| Styrene-isoprene-styrene block copolymer | 22.5 | 22.5 | 22.5 | 22.5 | 22.5 | 22.5 |
| Polyisobutylene | 7.5 | 7.5 | 7.5 | 7.5 | 7.5 | 7.5 |
| Liquid paraffin | 37.1 | 32.1 | 51.0 | 41.0 | 36.0 | 31.0 |
| 1-menthol | 3.0 | 3.0 | 3.0 | 3.0 | 3.0 | 3.0 |
| Dibutyl hydroxytoluene | 0.5 | 0.5 | 0.5 | 0.5 | 0.5 | 0.5 |
| Total | 100.0 | 100.0 | 100.0 | 100.0 | 100.0 | 100.0 |

### [Comparative Examples]

Nonaqueous preparations for percutaneous absorption with the compositions shown in Table 2 were manufactured by the same procedure as in the Examples. The amounts shown in Table 2 are in % by mass.

**[Table 2]**

| | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 |
|---|---|---|---|---|---|---|---|---|---|
| Loxoprofen sodium hydrate | 1.1 | 2.3 | 2.3 | 2.3 | 4.5 | 4.5 | 4.5 | 5.7 | 5.7 |
| Hydrogenated rosin glycerin ester | 20.0 | 0.0 | 5.0 | 40.0 | 0.0 | 5.0 | 40.0 | 10.0 | 20.0 |
| Phosphoric acid | 1.0 | 1.0 | 1.0 | 1.0 | 1.0 | 1.0 | 1.0 | 1.0 | 1.0 |
| Styrene-isoprene-styrene block copolymer | 22.5 | 22.5 | 22.5 | 22.5 | 22.5 | 22.5 | 22.5 | 22.5 | 22.5 |
| Polyisobutylene | 7.5 | 7.5 | 7.5 | 7.5 | 7.5 | 7.5 | 7.5 | 7.5 | 7.5 |
| Liquid paraffin | 44.4 | 63.2 | 58.2 | 23.2 | 61.0 | 56.0 | 21.0 | 49.8 | 39.8 |
| 1-menthol | 3.0 | 3.0 | 3.0 | 3.0 | 3.0 | 3.0 | 3.0 | 3.0 | 3.0 |
| Dibutyl hydroxytoluene | 0.5 | 0.5 | 0.5 | 0.5 | 0.5 | 0.5 | 0.5 | 0.5 | 0.5 |
| Total | 100.0 | 100.0 | 100.0 | 100.0 | 100.0 | 100.0 | 100.0 | 100.0 | 100.0 |

### [Test Example 1: Silicone Membrane Penetration Test]

A silicone membrane (thickness: 0.2 mm) cut into 2.5 cm in length and width was placed in a Franz diffusion cell for penetration test in which warm water at 32°C was circulated. A nonaqueous preparation for percutaneous absorption manufactured in Examples or Comparative Examples was cut into a circle with a diameter of 1 cm (effective area of 0.785 cm²) to obtain a specimen. The polyethylene terephthalate film (release liner) was peeled off from the specimen to expose the adhesive layer, and the adhesive layer was affixed to the upper portion of the silicone membrane to start the test. Underside of the silicone membrane was filled with Tyrode's solution (4 mL) as a receptor fluid, which was stirred with a magnetic stirrer until the end of the test. Twenty-four hours after the test started, 0.5 mL of the receptor fluid was collected, and the amount of drug contained was measured by high-performance liquid chromatography (HPLC). From the measured value, a 24-hour cumulative penetration amount (nmol/cm²) was calculated.

### [Test Example 2: Adhesion Test]

The nonaqueous preparations for percutaneous absorption obtained in Examples and Comparative Examples were evaluated by a finger tack test (after peeling off the release liner and pressing a finger onto the surface of the adhesive layer for 1 to 2 seconds, the adhesion when pulling the finger away from the surface was evaluated) according to the following criteria.

### Evaluation criteria

A: The adhesion is extremely good.
B: The adhesion has no problem with the use in a nonaqueous preparation for percutaneous absorption.
C: The adhesion is inadequate.

### [Test Example 3: Manufacturing Suitability Test]

The manufacturing suitability was evaluated according to the following criteria when each Example and each Comparative Example were manufactured.

### Evaluation criteria

A: The adhesive layer forming composition can be easily and uniformly stirred and spread.
B: The adhesive layer forming composition can be uniformly stirred and spread, although it is slightly stressful.
C: It is difficult to uniformly stir and spread the adhesive layer forming composition.

The results of Test Example 1 to Test Example 3 are shown in Table 3.

**[Table 3]**

| | Content of loxoprofen sodium (% by mass) *1 | Content of hydrogenated rosin glycerin ester (% by mass) | 24-hour cumulative penetration amount (mnol/cm²) | Adhesion *2 | Manufacturing suitability *3 |
|---|---|---|---|---|---|
| Comparative Example 1 | 1.0 | 20.0 | 500.7±4.2 | A | B |
| Comparative Example 2 | 2.0 | 0.0 | 831.4±17.4 | c | c |
| Comparative Example 3 | 2.0 | 5.0 | 927.8±12.1 | c | c |
| Example 1 | 2.0 | 10.0 | 996.8±11.0 | B | B |
| Example 2 | 2.0 | 20.0 | 1031.8±50.6 | A | B |
| Example 3 | 2.0 | 30.0 | 796.0±13.0 | A | B |
| Comparative Example 4 | 2.0 | 40.0 | 483.1±4.6 | A | A |
| Example 4 | 3.0 | 10.0 | 1255.8±44.9 | B | B |
| Example 5 | 3.0 | 15.0 | 1246.5±12.6 | B | B |
| Example 6 | 3.0 | 20.0 | 1174.0±38.5 | A | B |
| Example 7 | 3.0 | 25.0 | 1133.4±7.3 | A | B |
| Example 8 | 3.0 | 30.0 | 986.3±25.3 | A | B |
| Comparative Example 5 | 4.0 | 0.0 | 845.1±21.3 | c | c |
| Comparative Example 6 | 4.0 | 5.0 | 926.4±10.6 | c | c |
| Example 9 | 4.0 | 10.0 | 1070.1±26.0 | B | B |
| Example 10 | 4.0 | 20.0 | 882.6±17.1 | A | B |
| Example 11 | 4.0 | 25.0 | 918.5±14.8 | A | B |
| Example 12 | 4.0 | 30.0 | 807.5±7.5 | A | B |
| Comparative Example 7 | 4.0 | 40.0 | 644.2±15.1 | A | A |
| Comparative Example 8 | 5.0 | 10.0 | 777.2±60.5 | B | B |
| Comparative Example 9 | 5.0 | 20.0 | 672.3±14.3 | A | B |

| | | | | | |
|---|---|---|---|---|---|
| *1: The content of loxoprofen sodium is calculated on an anhydrous basis. *2: Evaluation criteria for adhesion A: The adhesion is extremely good. B: The adhesion has no problem with the use in a nonaqueous preparation for percutaneous absorption. C: The adhesion is inadequate. *3: Evaluation criteria for manufacturing suitability A: The adhesive layer forming composition can be easily and uniformly stirred and spread. B: The adhesive layer forming composition can be uniformly stirred and spread, although it is slightly stressful. C: It is difficult to uniformly stir and spread the adhesive layer forming composition. | | | | | |

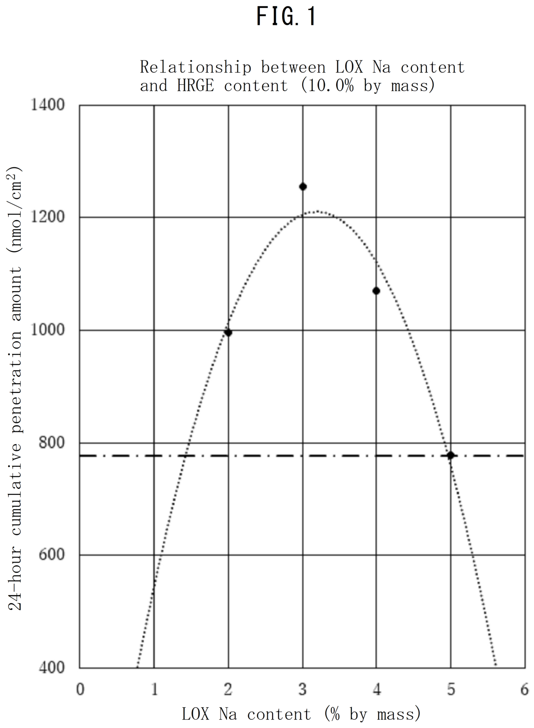

Regarding the silicone membrane penetration test, Fig. 1 graphically shows the results for Example 1, Example 4, and Example 9 and the results for Comparative Example 8 in Table 3. Comparative Example 8 is equivalent to Example 2 described in Patent Document 1.

From the results shown in Fig. 1, significant differences were observed between the 24-hour cumulative penetration amounts of Example 1, Example 4, and Example 9 and the 24-hour cumulative penetration amount of Comparative Example 8 when the content of the hydrogenated rosin glycerin ester (hereinafter, also referred to as "HRGE") was 10.0% by mass. The contents of the loxoprofen sodium (hereinafter, also referred to as "LOX Na") in Example 1, Example 4, and Example 9 were less than the content in Comparative Example 8 (the contents in Example 1, Example 4, and Example 9 were 2.0% by mass, 3.0% by mass, and 4.0% by mass, respectively, while the content in Comparative Example 8 was 5.0% by mass). However, the nonaqueous preparations for percutaneous absorption of Example 1, Example 4, and Example 9 had excellent skin penetration compared with the nonaqueous preparation for percutaneous absorption of Comparative Example 8.

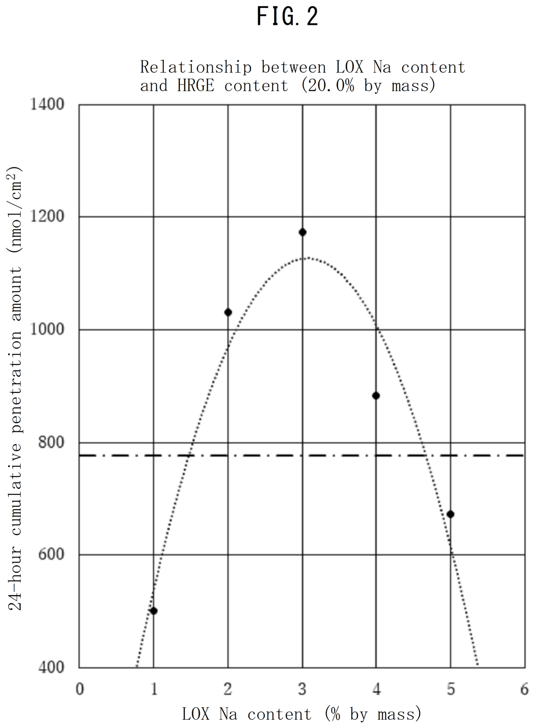

Regarding the silicone membrane penetration test, Fig. 2 graphically shows the results for Example 2, Example 6, and Example 10 and the results for Comparative Example 1 and Comparative Example 9 in Table 3. In Fig. 2, the long-chain line (- · -) is a line showing the 24-hour cumulative penetration amount in Comparative Example 8.

From the results shown in Fig. 2, significant differences were observed between the 24-hour cumulative penetration amounts of Example 2, Example 6, and Example 10 and the 24-hour cumulative penetration amount of Comparative Example 9 when the HRGE content was 20.0% by mass. Similarly to the results shown in Fig. 1, the contents of the LOX Na in Example 2, Example 6, and Example 10 were less than the content in Comparative Example 9 (the contents in Example 2, Example 6, and Example 10 were 2.0% by mass, 3.0% by mass, and 4.0% by mass, respectively, while the content in Comparative Example 9 was 5.0% by mass). However, the nonaqueous preparations for percutaneous absorption of Example 2, Example 6, and Example 10 had excellent skin penetration compared with the nonaqueous preparation for percutaneous absorption of Comparative Example 9.

As described above, since significant skin penetration was observed when the LOX Na content was 2.0% by mass to 4.0% by mass, the relationship between the LOX Na content (2.0% by mass to 4.0% by mass) and the HRGE content was examined.

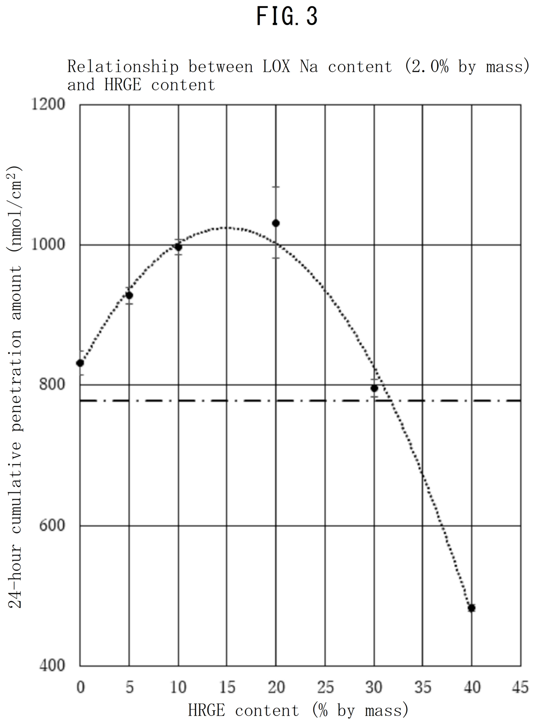

Regarding the silicone membrane penetration test, Fig. 3 graphically shows the results for Example 1 to Example 3 and the results for Comparative Example 2 to Comparative Example 4 in Table 3. In Fig. 3, the long-chain line (- · -) is a line showing the 24-hour cumulative penetration amount in Comparative Example 8.

From the results shown in Fig. 3, when the LOX Na content was 2.0% by mass, the 24-hour cumulative penetration amount decreased rapidly when the HRGE content was more than 30.0% by mass. In addition, from the results shown in Table 3, the adhesion and manufacturing suitability were inadequate in Comparative Example 2 and Comparative Example 3, whose HRGE contents were equal to or less than 5.0% by mass, and the nonaqueous preparations for percutaneous absorption of Comparative Example 2 and Comparative Example 3 did not have sufficient adhesion or manufacturing suitability.

Therefore, it is apparent from the results shown in Fig. 3 and Table 3 that when the LOX Na content is 2.0% by mass and the HRGE content is 10.0% by mass to 30.0% by mass, excellent skin penetration and sufficient adhesion and manufacturing suitability develop.

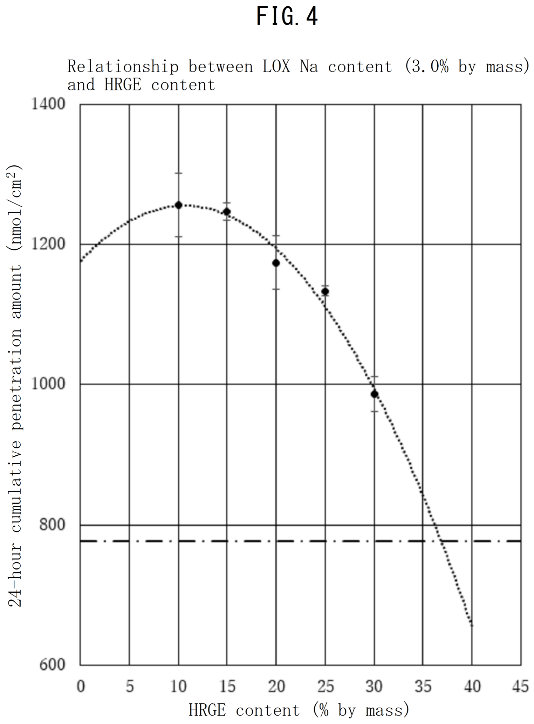

Regarding the silicone membrane penetration test, Fig. 4 graphically shows the results for Example 4 to Example 8 in Table 3. In Fig. 4, the long-chain line (- · -) is a line showing the 24-hour cumulative penetration amount in Comparative Example 8.

From the results shown in Fig. 4, when the LOX Na content was 3.0% by mass, significant differences were observed between the 24-hour cumulative penetration amounts of Example 4 to Example 8 and the 24-hour cumulative penetration amount of Comparative Example 8 when the HRGE content was 10.0% by mass to 30.0% by mass, and the nonaqueous preparations for percutaneous absorption of Example 4 to Example 8 had excellent skin penetration compared with the nonaqueous preparation for percutaneous absorption of Comparative Example 8.

Therefore, it is apparent from the results shown in Fig. 4 and Table 3 that when the LOX Na content is 3.0% by mass and the HRGE content is 10.0% by mass to 30.0% by mass, excellent skin penetration and sufficient adhesion and manufacturing suitability develop.

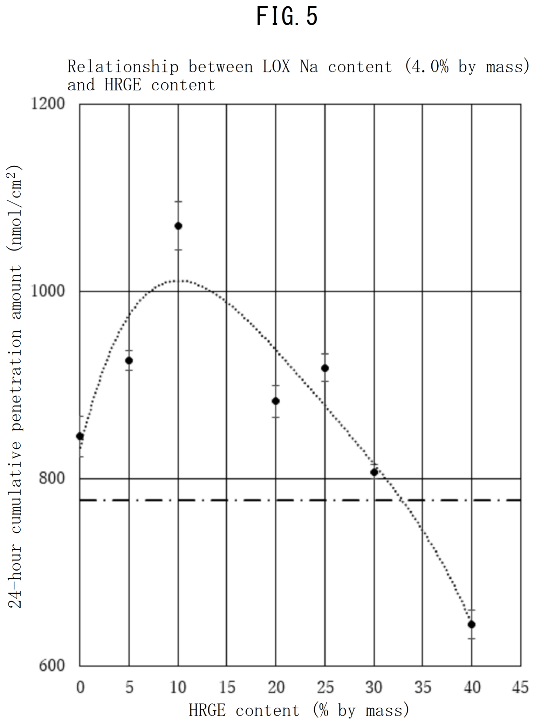

Regarding the silicone membrane penetration test, Fig. 5 graphically shows the results for Example 9 to Example 12 and the results for Comparative Example 5 to Comparative Example 7 in Table 3. In Fig. 5, the long-chain line (- · -) is a line showing the 24-hour cumulative penetration amount in Comparative Example 8.

From the results shown in Fig. 5, when the LOX Na content was 4.0% by mass, the 24-hour cumulative penetration amount decreased rapidly when the HRGE content was more than 30.0% by mass. In addition, from the results shown in Table 3, the adhesion and manufacturing suitability were inadequate in Comparative Example 5 and Comparative Example 6, whose HRGE contents were equal to or less than 5.0% by mass, and the nonaqueous preparations for percutaneous absorption of Comparative Example 5 and Comparative Example 6 did not have sufficient adhesion or manufacturing suitability.

Therefore, it is apparent from the results shown in Fig. 5 and Table 3 that when the LOX Na content is 4.0% by mass and the HRGE content is 10.0% by mass to 30.0% by mass, excellent skin penetration and sufficient adhesion and manufacturing suitability develop.

From these results, it is apparent that when the LOX Na content is 2.0% by mass to 4.0% by mass and the HRGE content is 10.0% by mass to 30.0% by mass, excellent skin penetration and sufficient adhesion and manufacturing suitability develop.

## Claims

1. nonaqueous preparation for percutaneous absorption comprising a support and an adhesive layer laminated on the support, wherein
the adhesive layer contains:
a non-steroidal anti-inflammatory drug in an alkali metal salt form in an amount of 2% by mass to 4% by mass based on a total mass of the adhesive layer;
a rosin ester derivative in an amount of 10% by mass to 30% by mass based on the total mass of the adhesive layer; and
an inorganic acid.

2. The nonaqueous preparation for percutaneous absorption according to claim 1, wherein
the non-steroidal anti-inflammatory drug is loxoprofen sodium hydrate, and
the rosin ester derivative is hydrogenated rosin glycerin ester.

3. The nonaqueous preparation for percutaneous absorption according to claim 1 or 2, wherein
the inorganic acid is phosphoric acid.

4. The nonaqueous preparation for percutaneous absorption according to claim 3, wherein
the adhesive layer contains a rubbery adhesive, and
the rubbery adhesive is a styrene-isoprene-styrene block copolymer.

5. The nonaqueous preparation for percutaneous absorption according to claim 4, wherein
the adhesive layer further contains at least one selected from the group consisting of a plasticizer, a tackifier, a percutaneous absorption accelerator, and a stabilizer.

6. The nonaqueous preparation for percutaneous absorption according to claim 5, wherein
the nonaqueous preparation for percutaneous absorption further comprises a liner layer laminated on the adhesive layer.
